# EUROPÄISCHE PATENTANMELDUNG

(11) **EP 2 039 255 A1**
(43) Veröffentlichungstag der Anmeldung: **25.03.2009**
(21) Anmeldenummer: 07116475.0
(22) Anmeldetag: 14.09.2007
(51) Int. Cl.: A23L 1/29, A23L 1/09, A61K 9/00, A61K 9/20

(54) **Formulierungen von Nahrungsergänzungsmitteln und festen, sich im Mund auflösenden süßen Genußmitteln**

(71) Anmelder: BASF SE, 67056 Ludwigshafen (DE)
(72) Erfinder: Widmaier, Ralf, 67061 Ludwigshafen (DE); Fussnegger, Bernhard, 67489 Kirrweiler (DE)

(57) **Zusammenfassung**

Formulierungen von Nahrungsergänzungsmitteln und festen, sich im Mund auflösenden süßen Genußmitteln

Mittel für die Anwendung in Nahrungsergänzungsmitteln und sich im Mund auflösenden festen süßen Genussmitteln, welche neben mindestens einem Wirkstoff als Matrixkomponente eine Mischung aus
a)60 - 98 Gew.-% mindestens eines Zuckers oder Zuckeralkohols oder Mischungen davon,
b) 1 - 25 Gew.-% eines Sprengmittels,
c) 1 - 15 Gew.-% von wasserunlöslichen Polymeren,
d) 0 - 15 Gew.-% wasserlöslichen Polymeren, und
e) 0 - 15 Gew.-% weiterer pharmazeutisch üblichen Hilfsstoffen,

wobei die Summe der Komponenten a) bis e) 100 Gew.-% beträgt, enthalten.

## Beschreibung

Die vorliegende Erfindung betrifft die Verwendung von Formulierungen in Form von Agglomeraten für die Herstellung von Nahrungsergänzungs- und sich im Mund auflösenden süßen Genußmitteln , wobei die Agglomerate Zucker oder Zuckeralkohole, Sprengmittel und wasserunlösliche Polymere enthalten.

In der heutigen Zeit besteht ein zunehmendes Interesse an Nahrungsergänzungsmitteln, da oft durch die Ernährung nicht genügend wichtige Vitamine, Mineralien und sonstige Stoffe wie beispielsweise Antioxidantien aufgenommen werden können. Solche Nahrungsergänzungsmittel werden häufig in der Form von Kapseln angeboten. Nachteilig an Kapseln ist aber, dass man diese üblicherweise mit etwas Flüssigkeit schlucken muss.

Weiterhin werden heutzutage auch viele Wirkstoffe pflanzlicher Herkunft, die teilweise auch eine pharmazeutische Verwendung haben, als Nahrungsergänzungsmittel zur Verbesserung des allgemeinen Wohlgefühls eingesetzt.

Zwar werden auch eine Reihe solcher Präparate als Lutsch- oder Kautabletten angeboten, wobei aber das Mundgefühl oft noch nicht optimal ist. Ausserdem haben viele Stoffe pflanzlicher Herkunft einen bitteren Geschmack, der beim Lutschen oder Kauen eher unangenehm wirkt.

Schnell zerfallende Tabletten bestehen häufig aus Zucker und Zuckeralkoholen, Brausesystemen, mikrokristalliner Cellulose und anderen nicht wasserlöslichen Füllstoffen Calciumhydrogenphosphat, Cellulose-Derivaten, Maisstärke, oder Polypeptiden. Weiterhin kommen wasserlösliche Polymere, übliche Sprengmittel (quervernetztes PVP, Na- und Ca-Salz der quervernetzten Carboxymethylcellulose, Na-Salz der Carboxymethylstärke, niedrigsubstituierte Hydroxypropylcellulose L-HPC) und im wesentlichen anorganische wasserunlösliche Bestandteile (Kieselsäuren, Silikate, anorganische Pigmente) zum Einsatz. Weiterhin können die Tabletten auch Tenside enthalten.

In der WO 2003/051338 ist eine direkttablettierbare und gut verpressbare Hilfsstoffformulierung, welche Mannit und Sorbit enthält, beschrieben. Zunächst wird durch Lösen von Mannit und Sorbit in Wasser und anschließender Sprühtrocknung (gewöhnliche Sprühtrocknung und SBD-Verfahren) eine Hilfsstoffvormischung hergestellt. Dieser coprozessierten Mischung kann zusätzlich Mannit zugesetzt werden. Tabletten, welche zusätzlich Sprengmittel, Trennmittel, Pigment und einen Wirkstoff enthalten, sollen innerhalb von 60 Sekunden in der Mundhöhle zerfallen.

In der US 2002/0071864 A1 wird eine Tablette beschrieben, welche innerhalb von 60 Sekunden in der Mundhöhle zerfällt, und hauptsächlich aus einer physikalischen Mischung von sprühgetrocknetem Mannit und einem grobkörnigen quervernetztem Polyvinylpyrrolidon sowie einer begrenzten Auswahl an Wirkstoffen formuliert ist. Diese Tabletten besitzen eine Bruchfestigkeit von ca. 40N und erzeugen ein unangenehmes, sandiges Mundgefühl.

Gemäß der US 6,696,085 B2 soll ein Methacrylsäure-Copolymer Typ C als Zerfallsmittel eingesetzt werden. Das Methacrylsäure-Copolymer Typ C ist ein magensaftresistentes Polymer, welches im sauren pH-Bereich nicht löslich ist, im pH-Bereich von 7, wie er in der Mundhöhle vorliegt, aber wasserlöslich ist. Die Tabletten weisen neben einer niedrigen Bruchfestigkeit (<20N) eine hohe Friabilität (>7%) auf und beinhalten einen hohen Anteil im Bereich von 15Gew.-% eines grobkörnigen Sprengmittels. Sie besitzen folglich eine niedrige mechanische Festigkeit und erzeugen aufgrund des hohen Anteils an grobkörnigem Sprengmittel ein unangenehmes, sandiges Mundgefühl.

EP 0839526 A2 beschreibt eine pharmazeutische Darreichungsform bestehend aus einem Wirkstoff, Erythrit, kristalliner Cellulose und einem Sprengmittel. Weiterhin wird Mannit eingearbeitet und als Sprengmittel quervernetztes Polyvinylpyrrolidon verwendet, sodass eine physikalische Mischung entsteht. Die Tabletten sollen innerhalb von 60 Sekunden in der Mundhöhle zerfallen.

In der Anmeldung JP 2004-265216 wird eine im Mund innerhalb von 60 Sekunden zerfallende Tablette, bestehend aus einem Wirkstoff, einem wasserlöslichen Polyvinylalkohol-Polyethylenglykol-Copolymer, Zucker/Zuckeralkohol (Mannit) und Sprengmittel, beschrieben.

Die Matrixkomponenten auf Basis von Zuckeralkoholen, Sprengmitteln und unlöslichen Polymeren sind allgemein für pharmazeutische Anwendungen aus der WO 2007/071581 bekannt.

Aufgabe der vorliegenden Erfindung war es, Darreichungsformen für Nahrungsergänzungsmittel und süße Genussmittel zu finden, die ein angenehmes Mundgefühl hinterlassen, gewünschtenfalls im Mund schnell zerfallen, und mechanisch sehr stabil sind.

Demgemäß wurden Zubereitungen für die Herstellung von festen Darreichungsformen von Nahrungsergänzungsmitteln und festen sich im Mund auflösenden süßen Genussmitteln gefunden, welche als Matrixsubstanz Agglomerate enthaltend
a) 60 - 98 Gew.-% mindestens eines Zuckers oder Zuckeralkohols oder Mischungen davon,
b) 1 - 25 Gew.-% eines Sprengmittels,
c) 1 - 15 Gew.-% von wasserunlöslichen Polymeren,
d) 0 - 15 Gew.-% wasserlöslichen Polymeren, und
e) 0 - 15 Gew.-% weiterer pharmazeutisch üblichen Hilfsstoffen,
, wobei die Summe der Komponenten a) bis e) 100 Gew.-% beträgt, enthalten

Weiterhin wurden im Mund schnell zerfallende Darreichungsformen sowie Kau- und Lutschtabletten, enthaltend solche Zubereitungen, gefunden. Die Tabletten können im Mund oder in wässrigem Milieu innerhalb von 40 Sekunden, bevorzugt innerhalb von 30 Sekunden, besonders bevorzugt innerhalb von 20 Sekunden zerfallen. Die Darreichungsformen können in Form von Komprimaten, also als Tabletten oder Pastillen angewendet werden oder als Lollis ausgelget sein. Weiterhin ist auch die Anwendung von Granulaten als Sachets möglich.

Die Darreichungsformen enthalten als Wirkstoffe Substanzen, wie sie üblicherweise für Nahrungsergänzungsmittel und für bestimmte süße Genussmittel eingesetzt werden.

Als feste, sich im Mund auflösende süße Genussmittel werden erfindungsgemäß Komprimate in Form von Pastillen oder Tabletten verstanden. Als Darreichungsformen eignen sich weiterhin Sachets und Lollis. Insbesondere eignen sich die Formulierungen auf dem Gebiet der sich im Mund auflösenden Genussmittel für Mittel zur schnellern Erfrischung des Atems. Die süßen Genussmittel zerfallen je nach Formulierung schnell und von selbst oder durch Lutschen oder Kauen.

Die Zubereitungen enthalten als Komponente a) 60 bis 98 Gew.-%, bevorzugt 70 bis 95 Gew.-%, besonders bevorzugt 75 bis 93 Gew.-% eines Zuckers, Zuckeralkohols oder Mischungen davon. Als Zucker oder Zuckeralkohole eignen sich Trehalose, Mannit, Erythrit, Isomalt, Maltit, Lactit, Xylit, Sorbit. Die Zucker- oder Zuckeralkoholkomponenten sind bevorzugt feinteilig, mit mittleren Teilchengrößen von 5 bis 100 µm. Gewünschtenfalls können die Teilchengrößen durch Mahlen eingestellt werden. Bevorzugte Teilchengrößen liegen bei 30 bis 50 µm. Es kann sich aber auch empfehlen Teilchengrößen kleiner 30 µm zu verwenden. Ebenso kann es sich empfehlen, Zucker beziehungsweise Zuckeralkohole einzusetzen, die Mischungen von Fraktionen mit unterschiedlicher Partikelgröße enthalten, beispielsweise Mischungen aus 30 bis 70 Gew.-% einer Korngrößenfraktion mit einer mittleren Teilchengröße von < 30 µm und 30 bis 70 Gew.-% einer Korngrößenfraktion mit einer mittleren Teilchengröße von 30 bis 50 µm. Bevorzugt werden Mannit, Erythrit oder Mischungen davon eingesetzt.

Als Komponente b) werden Sprengmittel in Mengen von 1 bis 25 Gew.-%, bevorzugt, 2 bis 15 Gew.-%, besonders bevorzugt 3 bis 10 Gew.-%, eingesetzt. Solche Sprengmittel sind wasserunlöslich, aber nicht filmbildend. Als Sprengmittel eignet sich Crospovidon, ein quervernetztes wasserunlösliches Polyvinylpyrrolidon, ebenso wie Croscarmellose, eine quervernetzte Carboxymethyllcellulose, wobei als Croscarmellose erfindungsgemäß auch deren Natrium- und Calciumsalze gemeint sind. Weiterhin eignet sich Natriumcarboxymethylstärke. Ebenso eignet sich L-Hydroxypropylcellulose, bevorzugt mit 5 bis 16% Hydroxypropoxy-gruppen.

Als Komponente c) werden wasserunlösliche Polymere eingesetzt in Mengen von 1 bis 15 Gew.-%, bevorzugt 1 bis 10 Gew.-%, eingesetzt. Dabei handelt es sich um Polymere. Bevorzugt sind Polymere, die im pH-Bereich von 1 bis 14 unlöslich sind, also eine bei jedem pH-Wert pH-unabhängige Wasserunlöslichkeit aufweisen. Weiterhin eignen sich aber auch Polymere, die bei jedem pH-Wert im pH-Bereich von 6 bis 14 wasserunlöslich sind.

Die Polymere sollen filmbildende Polymere sein. Filmbildend bedeutet in diesem Zusammenhang, dass die Polymere in wässriger Dispersion eine Mindestfilmbildetemperatur von -20 bis +150 °C, bevorzugt 0 bis 100 °C aufweisen.

Geeignete Polymere sind Polyvinylacetat, Ethylcellulose, Methylmethacrylat-Ethylacrylat-Copolymere, Ethylacrylat-Methylmethacrylat-Trimethylammoniumethylmethacrylat-Terpolymere. Butylmethacrylat-Methylmethacrylat-Dimethylaminoethylmethacrylat-Terpolymere Die Acrylat-Methacrylat-Copolymere sind näher beschrieben in der Europäischen Pharmacopoeia als Polyacrylate Dispersion 30%, in der USP als Ammonio Methacrylate Copolymer und in JPE als Aminoalkyl-Methacrylate Copolymer E. Als bevorzugte Komponente c) kommt Polyvinylacetat zum Einsatz. Dieses kann als wässrige Dispersion mit Feststoffgehalten von 10 bis 45 Gew.-% eingesetzt werden. Bevorzugt ist zudem Polyvinylacetat mit einem Molekulargewicht zwischen 100.000 und 1.000.000 Dalton besonders bevorzugt zwischen 200.000 und 800.000 Dalton.

Weiterhin können die Formulierungen als Komponenten d) wasserlösliche Polymere in Mengen von 0 bis 15 Gew.-% enthalten. Geeignete wasserlösliche Polymere sind beispielsweise Polyvinylpyrrolidone, Vinylpyrrolidon-Vinylacetat-Copolymere, Polyvinylalkohole, Polyvinylalkohol-Polyethylenglykol-Pfropfcopolymere, Polyethylenglykole, Ethylenglykol-Propylenglykol-Blockcopolymere, Hydroxypropylmethylcellulose, Hydroxypropylcellulose, Hydroxyethylcellulose, Carragenane, Pektine, Xanthane, Alginate. Gewünschtenfalls können durch Zusatz von üblichen Hilfsstoffen (Komponenten e)) in Mengen von 0 bis 15 Gew.-%, beispielsweise wie Säuerungsmitteln, Puffersubstanzen, Süßstoffen, Aromen, Geschmacksverstärkern und Farbstoffen Geschmack und Aussehen der aus den Formulierungen erhaltenen Tabletten weiter verbessert werden. Folgende Stoffe sind hierbei besonders geeignet:
Säuerungsmittel wie Citronensäure, Weinsäure, Ascorbinsäure, ÄpfelsäureNatriumdihydrogenphosphat,
Süßstoffe wie Cyclamat, Saccharin-Na, Aspartam, Acesulfam, Neohesperidin, Sucralose, Stevitidin

Aromostoffe wie: Menthol, Pfefferminzaroma, Fruchtaromen, wie Erdbeer, Johannisbeere, Wildkirsche, Apfel, Guave, Mango, Brombeere, Cranberry, Pfirsich, Maracuja, Zitrone, Orange, Mandarine. Grapefruit, Kakaoaroma, Vanillearoma, Glutamat,.

Farbstoffe wie Riboflavin, Betacarotin, wasserlösliche Farbstoffe, feinteilige Farblacke. Durch Zusatz von Verdickungsmitteln wie hochmolekularen Polysacchariden kann das Mundgefühl durch Erhöhung der Weichheit und des Volumengefühls zusätzlich verbessert werden.

Weiterhin können als Komponenten e) auch Tenside zugegeben werden. Als Tenside eignen sich beispielsweise Natriumlaurylsulfat, Dioctylsulfosuccinat, alkoxilierte Sorbitanester wie Polysorbat 80, polyalkoxilierte Derivate von Rizinusöl oder hydriertem Rizinusöl, beispielsweise Cremophor® RH 40, alkoxilierte Fettsäuren, alkoxilierte Hydroxyfettsäuren, alkoxilierte Fettalkohole, Alkalisalze von Fettsäuren und Lecithine. Weiterhin eignet sich Natriumstearylfumarat.

Weiterhin können zur weiteren Verbesserung des Zerfalls auch feinteilige Pigmente zugegeben werden, weil sie die inneren Grenzflächen erhöhen und somit Wasser schneller in die Tablette eindringen kann. Diese Pigmente wie Eisenoxide, Titandioxid, kolloidale oder gefällte Kieselsäure, Calciumcarbonate, Calciumphosphate müssen natürlich sehr feinteilig sein, ansonsten entsteht wiederum ein körniger Geschmack.

Die Herstellung der erfindungsgemäßen Formulierungen kann durch Aufbau-Agglomeration in Mischern, Wirbelschichtgeräten oder Sprühtürmen erfolgen. Dabei werden feste Ausgangsmaterialien und Granulierflüssigkeit zunächst miteinander vermischt und das feuchte Mischgut anschliessend getrocknet. Gemäß der vorliegenden Erfindung wird als Granulierflüssigkeit eine wässrige Dispersion der Komponente c), des wasserunlöslichen Polymers, eingesetzt.

Bei der Agglomeration in der Wirbelschicht wird eine wässrige Dispersion des wasserunlöslichen Polymers auf eine wirbelnde Mischung aus Zucker oder Zuckeralkohol und quervernetztem PVP gesprüht, wodurch die feinen Teilchen agglomerieren. Die Zulufttemperaturen betragen 30 bis 100°C, die Ablufttemperaturen 20 bis 70°C.

Bei der Herstellung in Sprühtürmen wird vorzugsweise die sogenannte FSD- oder SBD-Technologie (FSD: Fluidized spray drying; SBD: Spray bed drying) eingesetzt. Hierbei wird eine Lösung des Zuckers oder Zuckeralkohols in Wasser zunächst sprühgetrocknet, im unteren Teil des Sprühtrockners oder in einem angeschlossenen Wirbelbett erfolgt die Zugabe von quervernetztem PVP und die Eindüsung einer wässrigen Dispersion des wasserunlöslichen Polymeren, wodurch die Teilchen agglomerieren. Feine Teilchen können ferner nochmals vor die Sprühdüse der Zucker oder Zuckeralkohollösung geblasen werden und zusätzlich agglomeriert werden. Es ist im Sprühturm, FSD oder SBD auch eine Prozeßführung ausgehend von der kristallinen Form des Zuckers oder Zuckeralkohols möglich. Dabei wird der kristalline Zucker oder Zuckeralkohol am Kopf des Sprühturms oder in den Feingut-Recyclingstrom zugegeben. Durch das Versprühen einer wässrigen Dispersion des wasserunlöslichen Polymeren wird dieser kristalline Feststoff im Turm agglomeriert.

Für den Agglomerationsprozess kann es sich als günstig erweisen, einen mehrstufigen Sprühprozess zu fahren. Zu Anfang wird die Sprührate niedrig gehalten, um ein Überfeuchten der Produktvorlage und damit deren Verkleben zu verhindern. Mit zunehmender Prozessdauer kann die Sprührate erhöht und damit die Agglomerationstendenz erhöht werden. Es ist ebenfalls möglich die Zuluftmenge und/oder-temperatur in entsprechender Weise während des Prozesses anzupassen. Besonders während der Trocknungsphase ist es vorteilhaft, die Zuluftmenge zu reduzieren und damit einem Abrieb der Agglomerate durch eine hohe mechanischen Belastung vorzubeugen.

Als weitere Anpassungsparameter für die Agglomeratgröße kann die Feinheit des Sprühtröpfchens der Bindemittellösung bzw. -dispersion (einstellbar über den Zerstäubungsgasdruck), die Düsengeometrie und Abstand der Düse zum Produktbett angesehen werden. Je feiner und einheitlicher gesprüht wird, desto feiner und einheitlicher resultieren die Agglomerate. Je weiter die Düse vom Produktbett entfernt ist, desto schlechter ist das Agglomerationsverhalten.

Weiterhin können die Agglomerate auch in einem Mischer durch eine kontinuierlich geführte Mischaggregation erfolgen. Eine solche kontinuierlich geführte Form der Mischaggregation ist die sogenannte "Schugi-Granulation". Dabei werden in einem kontinuierlich arbeitenden vertikal angeordneten Hochgeschwindigkeitsmischer feste Ausgangsmaterialien und die das wasserunlösliche Polymer enthaltende Granulierflüssigkeit intensiv miteinander vermischt (siehe auch M. Bohnet, "Mechanische Verfahrenstechnik", Wiley VCH Verlag, Weinheim 2004, S. 198 ff.)

Gemäß einer besonderen Ausführungsform wird das quervenetzte PVP in der wässrigen Dispersion des wasserunlöslichen Polymers suspendiert.

Die so erhaltenen Agglomerate weisen eine mittlere Teilchengröße von 70 - 600 µm, bevorzugt 120 - 500 µm und besonders bevorzugt von 140 - 400 µm auf.
Das wasserunlösliche, filmbildende Polymer dient dabei als Agglomerationsmittel, um die feinen Zucker oder Zuckeralkoholkristalle und die Teilchen von Sprengmittel zu agglomerieren.

Die erfindungsgemäßen Formulierungen können vorteilhaft auch für die Herstellung von Tabletten verwendet werden, die vor der Anwendung in einem Glas Wasser zerfallen gelassen werden. Auch die Herstellung von Tabletten, die intakt geschluckt werden, ist natürlich möglich.

Für die Herstellung von Tabletten können die üblichen Verfahren verwendet werden, wobei die Direkttablettierung und die Walzenkompaktierung besondere Vorteile bieten. Aufgrund der besonderen Eigenschaften der erfindungsgemäßen Formulierungen werden in der Regel nur Wirkstoff, erfindungsgemäße Formulierung und ein Schmiermittel benötigt. Die Tablettenrezeptur ist somit sehr einfach, sehr reproduzierbar und das Verfahren leicht zu validieren.

Geeignete Wirkstoffe sind beispielsweise die Vitamine:
Vitamin A, Vitamin E, Vitamin D2, Vitamin D3, Vitamin B1, Vitamin B2, Vitamin B3 (Niacin) , Vitamin B5 (Pantothensäure), Vitamin B6, Vitamin B9 (Folsäure), Vitamin B12, Vitamin C, Vitamin K1 und K2, Biotin
Weiterhin eigenen sich: Coenzym Q10, Lutein, β-Carotin, Zeaxanthin, Lycopin, Quercetin

Ebenso eignen sich Mineralstoffe, die üblicherweise in Form ihrer physiologisch verträglichen wasserlöslichen Salze zum Einsatz kommen, beispielsweise Kalium-, Natrium-, Eisen(II)-, Magnesium-, Calcium-, Zink-, Kupfer(II)-, Mangan(II)-, Cobalt(II)- salze sowie Selen in Form von Salzen des Selenits, vorzugsweise Natriumselenit und weiterhin Jodid, Fluorid.
Geeignete Salze sind wasserlösliche Oxide, Chloride, Hydrochloride, Sulfate, Hydrogensulfate, Hydrogencarbonate, Carbonate, Ascorbate, Citrate, Lactate, Tartrate, Gluconate, Aspartate, Hydrogenaspartate, Glutamate, Hydrogenglucamate, Lysinate, Orotate, Lactobionate, 2-Aminoethyl-dihydrogenphosphate.

Beispielhaft seien genannt: Kaliumchlorid, Kaliumcitrat, Kaliumhydrogenaspartat, Natriumchlorid, Natriumcitrat, Natriumphosphat, Calciumcarbonat, Magnesiumcarbonat, Magnesiumcitrat, Magenesiumoxid, Eisen(II)chlorid, Eisen (II)gluconat, Eisen (II)hydrogenaspartat, Mangan(II)hydrogenaspartat, Zinkaspartat, Zinkoxid, Zinkorotat, Zinkgluconat, Zinksulfat,

Eingearbeitet wereden können auch probiotische Ingredientien, die durch schonendes Lyophilisieren und Zerkleinern des Lyophilisats bestimmter Bakterienkulturen erhalten werden können , beispielsweise von Stämmen der Gattung, Art und Varietäten von: Bifidobacterium animalis, bifidum, breve, infantis, adolescentis, lactis, longum; Lactobacillus acidophilus, helveticus, casei, casei Lafti, Casei Shirota, gasseri, johnsonii, rhamnosus, paracasei, plantarum, reuteri;
Streptococcus thermophilus.

Weiterhin eignensich Alpha-Liponsäure, Algenextrakte von Grün- oder Braunalgen, Arabinogalactan, Arginin, Baketerienlysate, Beta-alanin, Beta-sitosterol, Betain, Bieoflavonoide, Leucin, Isoleucin, Valin, Bromelain, Coffein, Carnitin, Casein-Proetinhydrolysat, Chitosan, Chlorella, Cholin, Chondroitin, Kakao-Flavanole, Kollagenhydrolysate, konjugierte Linolensäure, KreatinCystein-reiches ProteinHydrolysat, Docosahexaensäure, Epigallo-catechin-3-gallat, Omega-3-Fettsäuren, Fischöle, Muschelextrakte, insbesondere der neuseeländischen Grünlippmuschel, Glucomannan, Glucosamin, Glucuronolacton, Glutamin und Glutamin-Peptide, Lignan, Methionin, Papain, Phenylalanin, Phosphatidylcholin, Phosphatidylserin, Phytosterole (Mischung aus Beta-Sitosterol, Campesterol, Stigmasterol, Brassicasterol, Ergostanol, Campestanol), Kieselerde, Soja-isoflavone, Stearinsäure, Taurin oder Troxerutin, sowie Mischungen davon.

Hervorragend eignen sich die erfindungsgemäßen Formulierungen auch zur Verarbeitungen von Pflanzeninhaltsstoffen, deren nicht immer angenehmer Eigengeschmack übertönt wird. Geeignete Inhaltsstoffe Stoffe sind beipielsweise erhältlich aus: Ackergauchheil, Ackerhellerkraut, Ackerschachtelhalm,Ackerminze,Ackerschotendotter,Ackerstiefmütterchen,Ackerveilchen, Ackerwinde,Adonisröschen ,Akelei,Alant ,Allermannsharnisch,Aloe,Alraune,Ampfer,Ananas,Andorn,Angelika,Anis,Apfel,Arnika, Aronstab,Artischocke,Arzneikürbis,Augentrost, Bachbunge, Baldrian,Barbarakraut,Bärenklau,Bärentraube, Bärlapp, Bärlauch,Bartflechte,Basilikum,Basilienkraut,Beifuss,Beinwell,Belladonna,Benediktenkraut, Benzoe,Berberitze,Bergamotte,Berufkraut,Besenginster,Betelnusspalme,Betonie,Bibernell e, Bilsenkraut,Bingelkraut,Birke,Bitterholz,Bitterklee,Bittersüß,Blasentang,Blauhuder, Blutweiderich,Blutwurz,Bockshornklee, Bohnenkraut,Boldo,Braunwurz,Brechnuss, Brechwurzel,Breitwegerich,Brennessel,Brombeere,Bruchkraut,Brunnenkresse,Buche, Buchsbaum,Buchweizenkraut,Buckell,Buldermann,Cayennepfeffer, Chinarindenbaum, Christrose,Citronellgras,Dill, Dinkel,Diptam,Dost,Donnerrebe,Drachenblutbaum,Eberesche, Eberraute,Eberwurz,Edelgamander,EdelkastanieEfeu,Eibisch,Eiche,Einbeere, Eisenhut, Eisenkraut,Engelsüß,Engelwurz,Enzian,Erdbeere,Erdrauch,Esche,Espe,Estragon,Eukalyptu s, Farn,Färberginster,Faulbaum,Fenchel,Fettkraut,Fichte,Fingerhut, Roter,Fingerhut, Wolliger, Fliegenpilz,Flohsamen,Frauenmantel,Frauenminze,Fuchskreuzkraut, Fünffingerkraut, Galba-num,Galgant,Gamander,Gänseblume,Gänsefingerkraut,Gänsekraut,Gartenbohne,Gart enrose,Gartenschneeball,Gefleckter Schier-ling,Geissraute,Gelbholzbaum,Geranium,Gewürznelke, Giersch,Gingko,Ginseng, Gnadenkraut,Goldraute,Goldrute,Guarana,Gundelrebe,Gundermann,Günsel, Habichts-kraut,Hafer,Hagebutte,Hahnenfuß,Hamamelis,Haselnüsse,Haselwurz,Hauhechel, Haus-wurz,Heckenrose,Heidekraut,Heidelbeere,Herbstzeitlose,Herzgespann,Heublumen, Hibiskus,Himbeere,Hirtentäschel, Hirnkraut,Hohlzahn,Holunder,Honigklee,Hopfen,Huflattich,Immergrün, Ingwer,Iris,Irländisches Moos,Isländisches Moos,Jakobskreuzkraut,Johanniskraut,Johannisbeere,Josefskräutlein,Jungfernkraut, Kal-mus,Kamille,Kampfer,Kapuzinerkresse,Kardamom,Kartoffel,Kastanie,Katzenpfötchen, Kerbel,Kawa-Kawa,Kiefer,Klatschmohn,Klette,Knabenkraut,Knoblauch,Königskerze, Kohldistel,Koriander,Kornblume,Königskraut,Königsbalsam, Kreuzblume,Kreuzdorn Kümmel,Kuhschelle,Kurkuma,Kürbis,Labkraut,Lärche,Lavendel,Lebensbaum, Leberblümchen,Lein, Leinkraut,Liebstöckel,Linde,Löffelkraut,Löwenzahn,Lorbeer, Lungenkraut,Mädesüß,Mahonie,Mäuseklee,Maiglöckchen,Majoran,Malve,Mariendistel, Mäusedorn,Meerrettich,Meisterwurz,Melisse,Mistel,Möhre,Mönchspfeffer,Mugwurz, Mutterkorn,Mutterkraut,Myrrhe,Nachtkerze,Nelkenwurz,Niaouli,Niembaum,Nieswurz, Odermennig,Oleander,Oregano,Pappel,Paprika,Passionsblume,Pestwurz,Petersilie, Pfaffenhütchen,Pfefferkraut,Pfefferminze,Pfennigkraut, Pfingstrose,Preisselbeere, Quecke,Quendel,Rainfarn,Rauschbeere,Raute,Rauwolfia,Rhabarber,Rhododendron, Ringelblume,Rittersporn,Rizinus,Römische Kamille,Rosenöl,Rosmarin, Ruprechtskraut,Rosskastanie,Safran,Salbei,Sanddorn,Sandelholz,Sandsegge, Sanikel,Sassafras,Sauerampfer,Sägepalme,Sauerdorn,Sauerkraut,Schachtelhalm Schafgarbe ,Scharbockskraut,Schlafmohn ,Schlangenknöterich,Schlehdorn ,Schlüsselblume, Schmerwurz,Schnittlauch,Schöllkraut,Schwalbenwurz,Schwarzer Senf,Schwarzkümmel, Schwarztee,Schwertlilie,Seidelbast,Seifenkraut,Sellerie,Sennespflanze,Silberdistel, Sonnenblume,Sonnenhut,Sonnentau,Sonnwendgürtel,Spargel,Spitzwegerich,Springkraut, Stechapfel,Stechpalme,Steinklee,Sternanis,Wildes Stiefmütterchen,Stockrose, Storchenschnabel,Süßholz,Sumpfdotterblume,Taubnessel ,Tausendgüldenkraut, Teebaum,Teufelsabbiß,Teufelskerze,Teufelskralle,Thymian,Tollkirsche,Tormentill, Traubensilberkerze,Ulme,Uzara,Veilchen,Vogelknöterich, Wacholder, Walderdbeere,Waldmeister,Walnussbaum,Wasserdost (Wasserhanf, Kunigun-denkraut),Wasserminze,Wasserpfeffer,Wegwarte,Wehweide,Weide,Weidenröschen, Weihrauch,Weinrebe,Weissdorn,Weisskohl,Weisskraut,Wermut,Wicke, Wiesen-klee,Wiesenknopf Wiesenschaumkraut,Wilder Wermut, Wildro-se,Wintergrün,Wirsing,Wolfstrapp,Wundklee, Wundschwamm,Wurmfarn,Yams, Wilder Yams,Ysop,Zaunrübe, Zaunwinde, Ziest, Zimt, Zinnkraut, Zitronenbaum, Zitonenmelisse, Zitronenverbena, Zitwer, Zwergholunder, Zwetschge, Zwiebel.

Üblicherweise handelt es sich bei den pflanzlichen Inhaltsstoffen um gefriergetrocknete Extrakte von ethanolischen Auszügen. Im Einzelfall können auch getrocknete Ölextrakte eingesetzt werden.

Neben Extrakten von Heilkräutern können auch Gemüse- und/oder Obstextrakte verarbeitet werden.

Mit Extrakten pflanzlicher Stoffe lassen sich Darreichungsformen als Nahrungsergänzungsmittel zur Verbesserung des Wohlbefindens durch Eigenbehandlung der folgenden Indikationen herstellen

### Appetitanregung

Andorn, Angelikawurzel, Bockshornklee, Chinarinde, Enzianwurz, Hibiskus, Isländisch Moos, Kardamom, Löwenzahn, Koriander, Schafgarbe, Senfsamen, Wermutkraut, Zimt,

### Gefäße/Blutzirkulation

Ackerschachtelhalm, Bärlauch, Gingko biloba, Hamamelis, Knoblauch, Mäusedornwurzel, Rosskastanie, japanischer Schnurbaum, Süßklee, rotes Weinlaub Leberbeschwerden/ Galle Artischocke, Erdrauch, Knoblauch, Mariendiste, Ringelblume, Rosmarin

### Stoffwechsel

### Nachtkerze

### Immunsystem

Aloe vera, Anis, Brennessel, Camelia sinensis, Ginseng, Hagebutte, Holunderbeere, Hopfendolden, Ingwer, Kamille, Knoblauch, Kümmel, Kurkuma, Lindenblüten, Nachtkerze, Pfefferminze, Primelwurz, Sonnenhut, Sanddorn Sternanis, Süßholz, Taigawurzel, Teebaum, Thymian, Weißdorn, Zimt, Zitronengras

Herz/Kreislauf Artischocke, Bockshornklee, Brennessel, Camelia sinensis, Hibiskus, Ingwer, Knoblauch, Koriander, Nachtkerze, Olive, Weißdorn, Zitronengrass

### Lipidstoffwechsel

Artischocke, Camelia sinensis, Hopfendolden, Ilex, Zwiebel

### Verdauungssystem

### Aloe vera, Andorn,

Angelikawurzel, Anis, kanadisches Blutkraut, Cayenne Pfeffer, Chinarinde, Fenchel, Faulbaumrinde, Hagebutte, Hopfendolden, Ingwer, Isländisch Moos, Kamille, Kardamom, Koriander, Kümmel, Kurkuma, Löwenzahn, Malve, Pfefferminze, Ringelblume, Rosmarin, Salbei, Senna, Senfsamen, Sternanis, Süßholz, Wacholderbeere, Zimt, Zitronengrass, Zitronenverbene

### Glucosestoffwechsel

Aloe vera, Bockshornklee, Camelia sinensis, Eukalyptus, Ginseng, Holunderbeere, Olive, Zimt, Zwiebel

### Nieren/Harnwege

Ackerschachtelhalm, Bärentraubenblätter, Hagebutte, Mädesüß, Petersilie, kleinblütiges Weidenröschen, Kürbiskern, Löwenzahn, Wacholderbeere,

### Blut

Angelikawurzel, Brennessel, Lindenblüten,

### Hautbehandlung

### Aloe vera, Nachtkerze, Sanddorn

### Nerven/Entspannung

Baldrian, Hanf, Johanneskraut, Kamille, Kava kava, Knoblauch, Lindenblüten, Passionsblume, Pfefferminze, Rotbusch, Wacholderbeere, Zitronenverbene Bewegungsapparat/ Gelenke Brennessel, Camelia sinensis, Nachtkerze, Teufelskralle

### Atemwege

Andorn, Brennessel, Eukalyptus, Fenchel, Geranienwurzel, Hagebutte, Holunderbeere, Knöterich, Lindenblüten, Malve, Mädesüß, Schlüsselblume, Salbei, Sonnenhut, Spitzwegerich, Sternanis, Süßholz, Thymian,

### Antioxidantien

Camelia sinensis, Enzianwurz, Gingko biloba, Ginseng, Hagebutte, Holunderbeere, Kamille, Knoblauch, Kurkuma, Lindenblüten, Rosmarin, Salbei, Süßholz, Thymian, Weißdorn, Zistus, Zitronenmelisse, Zwiebel

### Gynäkologica

Angelikawurzel, Anis, Eisenkraut, Kümmel, Hopfendolden, Mönchspfeffer, Nachtkerze, Rotklee, Salbei, Traubensilberkerze,

### KognitivesSystem/ Anregung

Camelia sinensis, Enzianwurz, Gingko biloba, Ginsengwurzel, Guarana, Kakaobohne, Kaffeebohne, Kava kava, Rosmarin, Taigawurze; Zitronenmelisse

### Gewichtsreduktion

Camelia sinensis, Cayenne Pfeffer, Ilex

### Reisekrankheit

Ingwer

### Remineralisierung

Ackerschachtelhalm

Weiterhin eigen sich Extrakte aus Obst und Gemüse wie beispielsweise Äpfel, Orange, mandarine, Ananas, Kiwi, Preiselbeere, Pfirsich, Zitrone, Apfel, Limetten, Pomeranzen, Kapstachelbeere (Physalis) Papaya, Brombeere, Johannisbeere, Cranberry, Mango, Guave, Gapefruit, Weintrauben
Karotten, Petersilie, rote Bete, Brokkoli, Wirsing, Weißkohl, Spinat, Mangold,Grünkohl, Sauerampfer, Löwenzahn, Cilantro (Koriandergrün), Zitronengras, Pastinaken, Portulak, Brennessel.

Ebenso eigen sich Getreideprodukte wie Weizenkleie, Haferkleie, Qinoa, Dinkelkleie, Amaranth.

Weiterhin können die Darreichungsformen auch Gelatinepuilver oder Kieselerde enthalten.

Besonders bevorzugte Wirkstoffe für nahrungsergänzungsmittel sind β-carotin, Lycopin, Lutein, Coenzym Q10 sowie polyphenolhaltige Extrakte, beispielsweise aus Tee oder roten Weintrauben.

Bevorzugte süße Genussmittel sind im Mund schnell zerfallende Tabletten, die zur Atemerfrischung ätherische Öle wie Menthol und/oder Fruchtaromen, insbesondere Zitrusaromen, enthalten. Solche Darreichungsformen können für ein attraktiveres Aussehen auch künstliche oder natürliche Farbstoffe enthalten.

Die erfindungsgemäßen Zubereitungen weisen außergewöhnlich gute Fließfähigkeiten und Verpressbarkeiten auf, die zu mechanisch sehr stabilen Tabletten führen. Die Bruchfestigkeit der mit Hilfe der erfindungsgemäßen Formulierungen erzeugten Tabletten kann 30 N bis 120 N betragen. Häufig liegen die Bruchfestigkeiten oberhalb von 40 oder 50 N, selbst unter Verwendung schwer pressbarer Wirkstoffe. Bevorzugt sind 30 bis 80 N, besonders bevorzugt 30 bis 60 N. Die Friabilitäten betragen < 0,2 %. Beschädigungen beim üblichen Tablettenhandling treten somit nicht auf.

Die Auflösungsgeschwindigekit lässt sich über den Pressdruck steuern. Für sich spontan schnell auflösende Tabletten empfehlen sich niedrige bis mittlere Pressdrücke, für Kau- und Lutschtabletten mittlere bis höhere Pressdrücke.

### Beispiele

Die Darreichungsformen werden durch Vermischen und Verpressen der Pflanzenextrakte mit einer Tablettengrundmischung und gegebenenfalls weiteren üblichen Hilfsstoffen

Die Agglomerate wurden in der Wirbelschicht (GPCG 3.1, Glatt) mittels Top- SprayVerfahren hergestellt: Zuckeralkohol und Sprengmittel (Croscarmellose, quervernetzte Natriumcarboxymethylstärke, L-Hydroxypropylcellulose)
wurden vorgelegt und mit wässriger Bindemitteldispersion agglomeriert. Als wässrige Bindemitteldispersion wurde eine kommerziell erhältliche Polyvinylacetatdispersion (Kollicoat® SR30 D, Fa. BASF AG) verwendet. Als L-Hydroxypropylcellulose wurde eine Type mit einem Hydroxypropoxy-Gehalt von 11 % verwendet.

**Tabelle 1: Rezepturzusammensetzung in Gew.%**

| | A | B | C | D | E | F |
|---|---|---|---|---|---|---|
| Mannitol (d_{0,5}:36µm] | 90 | 90 | 90 | 44 | 76 | 90 |
| Erythritol (d_{0,5}:44µm] | | | | 44 | | |
| Sorbitol (d_{0,5}:47µm] | | | | | 11 | |
| Crospovidon | | | | | | 5 |
| Croscarmellose-Na | 5 | | | | | |
| Croscarmellose-Ca | | | | 8 | | |
| quervernetzte Natriumcarboxymethylstärke | | 5 | | | | |
| L-Hydroxypropylcellulose | | | 5 | | | |
| Kollicoat SR30D (Feststoff) | 5 | 5 | 5 | 4 | 6 | 5 |

In einem zweistufigen Agglomerationsprozess, bei dem zunächst eine geringere Sprührate gewählt wurde und dann die Sprührate erhöht wurde, wurden folgende Herstellungsparameter angewendet :

| | |
|---|---|
| Ansatzgröße: | 0,6 kg |
| Konzentration der Bindemitteldispersion: | 10 Gew.% Feststoff |

**Tabelle 2: Herstellungsbedingungen der Formulierungen**

| | A | B | C | D | E | F |
|---|---|---|---|---|---|---|
| Zulufttemperatur [°C] | 49 | 52 | 52 | 51 | 53 | 49 |
| Ablufttemperatur [°C] | 25 | 28 | 31 | 27 | 31 | 25 |

Die so hergestellten Agglomerate wurden mit 2,0 Gew.% Schmiermittel (Mg-Stearat) bezogen auf die zu verpressende Mischung im Turbulamischer gemischt und auf einer vollinstrumentierten Excenterpresse (Korsch XP1) bei 30Hüben/min zu Tabletten verarbeitet.

Zur Tablettierung wurde die Excenterpresse mit einem 10mm- Stempel (biplan, facettiert) ausgestattet und die Presskraft so eingestellt, dass Tabletten mit einer Bruchfestigkeit von 40-60N resultierten.

Die Tabletten wurden hinsichtlich Bruchfestigkeit (Tablettentester HT-TMB-Cl-12 F, Fa. Kraemer), Zerfallszeit in Phosphatpuffer pH 7,2 (Zerfallstester ZT 74, Erweka) und Freisetzungsgeschwindigkeit in Magensaft (Freisetzungsgerät, Erweka) untersucht.

**Tabelle 3: Tabletteneigenschaften**

| | Tablettierdaten | Tablettenparameter | |
|---|---|---|---|
| | Presskraft [kN] | Bruchfestigkeit [N] | Zerfallszeit [s] |
| A | 4,0 | 49 | 15 |
| B | 3,7 | 51 | 21 |
| C | 3,8 | 40 | 53 |
| D | 5,3 | 52 | 38 |
| E | 4,6 | 51 | 32 |
| F | 4.0 | 50 | 18 |

### Rezepturen

In den im folgenden genannten Beispielrezepturen wird eine Tablettengrundmasse aus Agglomeraten aus 98 Gew.-% einer der Formulierungen A bis F und 2 Gew.-% Mg-Stearat verwendet.

Pro Tablette Coenzym Q10 ... mg/ Vitamin E ..., Tablettengrundmasse ad 100 %

Lycopin 1 mg, Tablettengrundmasse ad 100 %

Zeaxanthin 1 mg, Tablettengrundmasse ad 100 %

### Frucht-Magnesium-Tabletten

### Säuerungsmittel: Zitronensäure

Magnesiumcarbonat entsprechend 300 mg Magnesium pro100 g Tablettengrundmasse, Fruchtpulver, Fruchtaroma (Johannisbeere, Wildkirsche, Orange)

Vitamin C-Tablette: 25 oder50 mg Vitamin C, Zinksulfat entsprechend 5 mg Zink, Tablettengrundmasse ad 100 %

Calcium-Tablette:
Pro Tablette 600 mg Calcium als Calciumcarbonat plus 5 µg Vitamin D3, Kakaoaroma, Tablettengrundmasse ad 100 %

### Salbei-Magnesium-Tablette: Gewicht 3.6 g

Pro 100 g Tablettiermischung : 97 g Tablettengundmischung, 440 mg Vit C, 0.08 g Süßstoffe: 0.03 Acesulfam-K, 0.05 Aspartam, Säuerungsmittel Zitronensäure, Salbeiöl, Tablettengrundmasse ad 100 %

### Brausetablette: Gewicht 4.65 g

### Säuerungsmittel: Zitronensäure, Äpfelsäure

### Natriumhydrogencarbonat, Natriumcarbonat,

Cyclamat; Saccharin, Vit B9 600 µg, Vit B6 6 mg Vit B12 5 µg, Tablettengrundmasse ad 100 %

Vitamin C-Erdbeer-Lollys: Gewicht 15 g; 15 mg Vit C plus Fruchtaroma, Tablettengrundmasse ad 100%

Gemüseergänzung:
50 Gew.-% ethanolischer Gemüseextrakt, gefriergetrocknet aus Karotten, Petersilie, rote Bete, Brokkoli, Wirsing, Weißkohl, Spinat, Zuckerrübenfasern, Gerstenkleie, Knoblauchpulver, Spirulina pacifica (Grünalgenart), 50 mg Vit C, 13 mg Niacin, 9 mg β-Carotin, 1.5 mg Vit B6, 1 mg B2, 0.6 mg B1, 300 µg Folsäure, Q10, 30 mg Vit E, Lactobacillus bifidus 1 mg, Tablettengrundmasse ad 100 %

Fruchtergänzung :
50 Gew.-% ethanolischer, gefriergetrocknetes Pulver
Äpfel, Orange, Ananas, Kiwi, Preiselbeere, Pfirsich, Orangenschalen,Gelatine, vit C, Apfelpektin, Limetten, Pomeranzen, Kapstachelbeere (Physalis)" Zitruspektin Papayapulver, Lactobacillus acidophilus gefriergetrocknet, Dattelpulver, Zwetschgenpulver, Dunaliella salina (Grünalgen), 50 mg Vit C, 13 mg Niacin, 9 mg β-Carotin, 1.5 mg Vit B6, 1 mg B2, 0.6 mg B1, 300 µg Folsäure, Q10, 30 mg Vit E 3 mg, Tablettengrundmasse ad 100 %

### Energie-Mix

50 Gew.-% ethanolischer Extrakt, gefriergetrocknet aus Sanddorn, Blutorange, Acerola, Brombeere, Birnendicksaft, Fenchel, Sellerie, Tablettengrundmasse ad 100 %

Zur Entspannung :
Öl von : Lavendel, Zitrone, Macis, Thymian, Nelke, Zimt
Kardamom, Muskat, Koriander, Tablettengrundmasse ad 100 %

Zur Erfrischung :
Ztronenverbena, Hagebutte, Hibiskus, Zitronenmelisse
Tablettengrundmasse ad 100 %

Zur Verdauungsförderung:
50 mg Extrakt aus Kümmel, Wermut, Baldrian, Tablettengrundmasse ad 100 %

### Weihnachtpastillen

Hibiskus, Kornblumenblüten, Sonnenblumenblüten, Zimt, Nelken, Sultaninen (gefriergetrocknet und gemahlen) Orangenschalen, Rumaroma, Tablettengrundmasse ad 100 %

### Ingwerpastillen

Extrakt aus Zitronengras, Ingwer, Brombeerblätter, Minze, Zimt, Süßholz, Kardamom, Tablettengrundmasse ad 100 %

### Verdaungsdrops

100 mg Dinkelkleie, 50 mg Pfefferminzöl, Tablettengrundmasse ad 100 %

### Kräftig und adstringierend

Hirtentäschel, , ethanol. Extrakt aus frischen grünen Blättern 50 mg, Tablettengrundmasse ad 100 %

Hustenpastillen: 50 mg Trockenextrakt aus Efeublättern, Auszugsmittel Ethanol, Tablettengrundmasse ad 100 %

### Hustenpastillen

Eucalyptusöl 11 mg, Tablettengrundmasse ad 100 %

### Hustenpastillen

10 mg Dickextrakt aus Thymiankraut, gefriergetrocknet, Tablettengrundmasse ad 100 %

### Zur Stärkung

10 mg Ginseng, Tablettengrundmasse ad 100 %

### Zur Immunstärkung

50 mg Extrakt von Pelargonium sidoides-Wurzeln, Tablettengrundmasse ad 100 %

### Erkältungspastillen

60 mg Primelwurzel Trockenextrakt, Tablettengrundmasse ad 100 %

50 mg Eucalyptusöl, Anisöl, Pfefferminzöl, Tablettengrundmasse ad 100 %

50 mg Extrakt Bitterer Fenchel, Schlüsselblume, Tablettengrundmasse ad 100 % Kastanienblätter, Schachtelhalmkraut, Spitzwegerichkraut, Primelwurzel, Wollblumenblüten, Thymian, Königskerze, Tablettengrundmasse ad 100 %

Hustentabletten: lpecacuanha D3 25 mg, Tablettengrundmasse ad 100 %

Halslutschpastillen: Isländisch MoosTrockenextrakt 125 mg, Tablettengrundmasse ad 100 %

700 mg Eukalyptusöl, 250 mg Cajeputöl, Tablettengrundmasse ad 100 %

Pflanzliche Arteriosklerosemittel :
100 mg Knoblauchzwiebelpulver, Tablettengrundmasse ad 100 %
100 g Trockenextrakt, ethanolischer Auszug aus Olivenblättern, Tablettengrundmasse ad 100 %
50 mg Knoblauchzwiebelpulver, 30 mg Weißdornfrüchtepulver 30 mg Mistelkrautpul-ver,Tablettengrundmasse ad 100 %

### Zur Leberstärkung

329-324 mg Trockenextrakt aus Mariendistelfrüchten, entsprechend 184 mg Silymarin , ethanol. Auszug, Tablettengrundmasse ad 100 %

350 mgTrockenextrakt aus Artischocken, Tablettengrundmasse ad 100 %
Anti-Knoblauch-Drops
10 mg Kardamompulver,
Tablettengrundmasse ad 100 %

### Zur Stärkung

Lutein 6mg, Heidelbeer-Extrakt 50 mg, Omega-3-Fettsäure 140 mg Vitamin A 1 mg, Vitamin E 3 mg,Vitamin B2 2 mg, Vitamin C 20 mg, Tablettengrundmasse ad 100 %

### Calcium-Lutschtablette

Pro Tablette 400 mg Calcium, Vitamin D3 2.5 µg

### Muschel-Konzentrat

350 mg Grünlippmuschel-Konzentrat
Vitaminamin E 3,3 mg
Folsäure 66 µg
Vitaminamin C 20 mg
Vitaminamin B6 0.67 mg
Vitaminamin B12 0.33 µg

### Algenextrakt

Spirulina platensis 400 mg, β-Carotin 0.2 mg, Tablettengewicht 500 mg, Tablettengrundmasse ad 100 %

### Zur Stärkung

Lecithin 230 mg, Vitamin B1 2 mg, Vitamin B2 2 mg, Vitamin B6 3 mg, Vitamin B12 1,5 µg, Folsäure300 µg, Tablettengrundmasse ad 100 %

Brennesselextrakt 200 mg, Vitamin C 10 mg, Tablettengrundmasse ad 100 %

### Vitaminamin-B-Komplex

Vitamin B1 4 mg, Vitamin B2 4 mg, Vitamin B6 6 mg, Vitamin B12 3 µg, Niacin 18 mg Pantothensäure 18 mg, Folsäurer 600 µg, Tablettengrundmasse ad 100 %

### Aloe-Vera Kautabletten

Aloe-Vera-Konzentrat, gefriergetrocknet 200 mg, Vitamin C 75 mg, Vitamin E 12 mg, Niacin 18 mg, Pantothensäure 6 mg, Vitamin B1 1.3 mg, Vitamin B2 1.5 mg, Vitamin B6 1.6 mg, Vitamin B12 3 µg, Folsäure 150 µg, Biotin 60 µg, Tablettengrundmasse ad 100 %

### MultiVitamin- und Mineral-Kautabletten

Pro Tablette Vitamin C 11 mg, Niacin 18 mg, Vitamin E 10 mg, Pantothensäure 6 mg, Vitamin B6 2 mg, Vitamin B2 1.6 mg, Vitamin B1 1.4 mg, Vitamin A 800 µg, Folsäure 200 µg, Biotin 150 µg, Vitamin K1 30 µg, Vitamin D3 5 µg, Vitamin B12 1 µg, Calcium 160 mg, Kalium 50 mg, Zink 5 mg, Eisen 4 mg, Kupfer 1 mg, Mangan 1 mg, Jod 100 µg, Chrom 60 µg, Selen 30 µg, Molybdän 25 µg , Tablettengrundmasse as 100 %

### Carnitin

pro Tablette L-Carnitin 300 mg, Vitamin C 20 mg, Tablettengrundmasse ad 100 % Biotin

pro Tablette 0.2 mg Biotin, Tablettengrundmasse ad 100 %

### Magnesium-Kautablette

Magnesium 60 mg, Vitamin C 30 mg pro Tablette, Tablettengrundmasse ad 100 %.

### Selen

Pro Tablette Vitamin C 50 mg, 6 mg, β-Carotin 2 mg, Selen 50 µg, Tablettengrundmasse ad 100 %

### Atemerfrischung

50 mg Mentol, kristallin, 20 mg Vitamin C, Tablettengrundmasse ad 100 %

### Atemerfrischung

50 mg Zitrusöl,Trockenpulver, Tablettengrundmasse ad 100 %

## Patentansprüche

1. Mittel für die Anwendung in Nahrungsergänzungsmitteln und sich im Mund auflösenden festen süßen Genussmitteln, welche neben mindestens einem Wirkstoff als Matrixkomponente eine Mischung aus
a)60 - 98 Gew.-% mindestens eines Zuckers oder Zuckeralkohols oder Mischungen davon,
b) 1 - 25 Gew.-% eines Sprengmittels,
c) 1 - 15 Gew.-% von wasserunlöslichen Polymeren,
d) 0 - 15 Gew.-% wasserlöslichen Polymeren, und
e) 0 - 15 Gew.-% weiterer pharmazeutisch üblichen Hilfsstoffen,
wobei die Summe der Komponenten a) bis e) 100 Gew.-% beträgt, enthalten.

2. Mittel nach Anspruch 1, enthaltend als Zuckeralkohol a) Mannitol.

3. Mittel nach Anspruch 1 oder 2, enthaltend als Komponente b) ein Polymer ausgewählt aus der gruppe bestehend aus quervernetztem Polyvinylpyrrolidon (Crospovidon), Croscarmellose, Natrium- und Calciumsalze der Croscarmellose, Natriumcarboxymethylstärke L-Hydroxypropylcellulose.

4. Mittel nach einem der Ansprüche 1 bis 3, enthaltend als Komponente b) Crospovidon.

5. Mittel nach einem der Ansprüche 1 bis 4, enthaltend als Komponente c) Polyvinylacetat, Ethylcellulose, Copolymere der (Meth)acrylsäure oder Mischungen davon.

6. Mittel nach einem der Ansprüche 1 bis 5, enthaltend als Komponente d) Polyvinylpyrrolidone, Vinylpyrrolidon-Vinylacetat-Copolymere, Polyvinylalkohole, Polyvinylalkohol-Polyethylenglykol-Pfropfcopolymere, Polyethylenglykole, Ethylenglykol-Propylenglykol-Blockcopolymere oder Mischungen davon.

7. Mittel nach einem der Ansprüche 1 bis 6, enthaltend als Komponente e) Hilfsstoffe wie Säuerungsmittel, Puffersubstanzen, Süßstoffe, Aromen, Geschmacksverstärker und Farbstoffe.

8. Mittel nach einem der Ansprüche 1 bis 7, enthaltend als Wirkstoff mindestens ein-Nahrungsergänzungsmittel.

9. Mittel nach einem der Ansprüche 1 bis 7, enthaltend als Wirkstoff mindestens einen Aromastoff.

10. Mittel nach einem der Ansprüche 1 bis 9, enthaltend als Wirkstoff ein oder mehrere Vitamine.

11. Mittel nach einem der Ansprüche 1 bis 10, enthaltend als Wirkstoff ein Carotinoide wie β-Carotin, Zeaxanthin, Lycopin, sowie Lutein oder Coenzym Q10.

12. Mittel nach einem der Ansprüche 1 bis 11, enthaltend als Wirkstoff einen Pflanzeextrakt.

13. Mittel nach einem der Ansprüche 1 bis 12, in Form einer durch Komprimierung erhaltenen Tablette oder Pastille.

14. Mittel nach einem der Ansprüche 1 bis 13, in Form von im Mund schnell zerfallenden Tabletten.

15. Mittel nach einem der Ansprüche 1 bis 14, in Form einer Lutsch- oder Kautablette.

16. Mittel nach einem der Ansprüche 1 bis 11, in Form eines Granulats.

17. Mittel nach einem der Ansprüche 1 bis 16, enthaltend einen oder mehrere Wirkstoffe in Mengen 0.001 bis 50 Gew.-%, bezogen auf das Gesamtgewicht des Mittels.

18. Verwendung von Mitteln nach einem der Ansprüche 1 bis 17, Nahrungsergänzungsmittel.

19. Verwendung von Mitteln nach einem der Ansprüche 1 bis 17 für sich im Mund auflösende feste süße Genussmittel , zur Behandlung von Mundgeruch, Karies, Zahnbelag, Zahnverfärbungen und mikrobiell verursachten Erkrankungen der Mund- und Rachenschleimhaut.

20. Verwendung nach Anspruch 19 für genussmittel zur Atemerfrischung.
